# EUROPEAN PATENT SPECIFICATION

(11) **EP 1 842 903 B1**
(45) Date of publication and mention of the grant of the patent: **11.02.2015**
(21) Application number: 06127219.1
(22) Date of filing: 27.12.2006
(51) Int. Cl.: C12M 1/33

(54) **Microfluidic device and method for concentrating a sample containing cells or viruses and lysing the cells or viruses, and method of producing the microfluidic device**
Mikrofluidische Vorrichtung und Verfahren zur Konzentration einer zellen- oder virenhaltigen Probe sowie zur Zell- oder Viruslyse und Verfahren zur Herstellung der mikrofluidischen Vorrichtung
Dispositif et procédé microfluide pour concentrer un échantillon contenant des cellules ou virus, et pour lyser les cellules ou virus, et procédé de fabrication du dispositif microfluide

(30) Priority: 07.04.2006 KR 20060031931
(43) Date of publication of application: 10.10.2007
(73) Proprietor: Samsung Electronics Co., Ltd., Suwon-si, Gyeonggi-do, 443-742 (KR)
(72) Inventor: Lee, Hun-joo, c/o Samsung Adv. Institute of Techn., Giheung-gu, Yongin-si, Gyeonggi-do (KR); Kim, Joon-ho, c/o Samsung Adv. Institute of Techn., Giheung-gu, Yongin-si, Gyeonggi-do (KR); Jeong, Sung-young, c/o Samsung Adv. Inst. of Tech., Giheung-gu, Yongin-si, Gyeonggi-do (KR); Hwang, Kyu-youn, c/o Samsung Adv. Inst. of Techn., Giheung-gu, Yongin-si, Gyeonggi-do (KR)
(74) Representative: Grünecker, Kinkeldey, Stockmair & Schwanhäusser

(56) References cited:
- EP-A- 1 672 459
- EP-A1- 1 541 234
- EP-A1- 1 672 059
- WO-A-02/12896
- WO-A2-2006/036307
- US-A1- 2003 075 446

## Description

### BACKGROUND OF THE INVENTION

### 1. Field of the Invention

The present invention relates to a microfluidic device for concentrating a sample containing cells or viruses and for lysing the cells or viruses, a method of producing the microfluidic device, and a method of concentrating a sample containing cells or viruses and lysing the cells or viruses using the microfluidic device.

### 2. Description of the Related Art

In general, biological analysis processes, such as the detection of pathogens and molecular diagnosis, consist of separation of target cells from a sample, concentration of a sample containing cells, separation and amplification of biomolecules from the cells, hybridization, and detection.

A lab-on-a-chip (LOC), which allows such a series of biological analysis processes to be rapidly and automatically performed on a microchip, is currently a subject that is being researched.

The LOC includes microfluidic devices in order to perform such biological analysis processes. A microfluidic device refers to a device in which inlets, outlets, reaction chambers and the like are fluidically connected through microchannels. Such microfluidic devices generally include, in addition to the microchannels formed thereon, micropumps for transferring a fluid, micromixers for mixing the fluid, microfilters for filtering the transferred fluid, and so on.

Conventional devices intending to integrate the biological analysis processes are composed of cell counting chambers, cell sorting chambers, DNA extraction chambers and PCR amplification chambers, wherein such chambers are fluidically connected in series by means of channels and respectively chambers are provided with valves and pumps.

EP1141234 refers to a microfluidic system comprising a circuit board with electrodes and a lacquer coating which is structure using photolithography or electron beams to leave the electrodes free. The lacquer consists of acrylic, epoxy, phenolic or silicone resins or fluoropolymer. A second layer is applied over this which has microchannels in its outer surface. This layer is made from SYLGARD (RTM; Dow Corning, polydimethylsiloxane), other organosiloxanes, their polymerization products, silicones, polyacrylates, e.g. polymethyl methacrylate, and/or elastomers with O- or N-containing functional groups, e.g. polysulfones, -imides, -carbonates and/or -acrylonitriles. The channels are in contact with the lacquer layer and bonded to it. The electrodes are positioned in the channels.

WO0212896 relates generally to the field of moiety or molecule manipulation in a chip format. In particular, the invention provides a method for manipulating a moiety in a microfluidic application, which method comprises: a) coupling a moiety to be manipulated onto surface of a binding partner of said moiety to form a moiety-binding partner complex; and b) manipulating said moiety-binding partner complex with a physical force in a chip format, wherein said manipulation is effected through a combination of a structure that is external to said chip and a structure that is built-in in said chip, thereby said moiety is manipulated.

However, in the case of constructing a device that simply integrates the biological analysis processes one behind the other in series, a large number of valves and microfluidic controllers are needed, thus making it difficult to integrate the biological analysis processes in a single device. Also, a large number of chambers is needed, thus making the volume of the device excessively large and costly. Moreover, there is a high possibility of a generation of air bubbles and a loss of samples during the transfer of sample solutions between the chambers.

Therefore, in order to miniaturize the LOC, as many biological analysis processes as possible are should be performed in a single chamber.

### SUMMARY OF THE INVENTION

The present invention provides a microfluidic device that allows formation of a concentrate of a sample containing cells or viruses and the lysis of the cells or viruses in a single chamber.

The present invention also provides a method of producing the microfluidic device.

The present invention also provides a method of simultaneously concentrating a sample containing cells or viruses, and lysing the cells or viruses in a single chamber of the microfluidic device.

According to an aspect of the present invention, there is provided a microfluidic device for concentrating cells from a sample containing cells or viruses and for lysing the cells or viruses, the device comprising an anode chamber containing an anode electrode; a cathode chamber containing a cathode electrode; and an ion exchange membrane separating the anode chamber and the cathode chamber, wherein a solid support capable of retaining cells or viruses from the sample containing cells or viruses is disposed in the cathode chamber, wherein the surface of the solid support is coated with a cell-binding substance, and when the solid support has a structure selected from the group consisting of a flat structure, a pillar structure, a bead structure and a sieve structure.

According to another embodiment of the present invention, the cell-binding substance is a hydrophobic substance having a water contact angle of 70° to 90°, or a charge donor substance.

According to another embodiment of the present invention, the hydrophobic substance is selected from the group consisting of octadecyltrichlorosilane (OTS), tridecafluorotetrahydrooctyl trimethoxysilane (DTS), octadecyldimethyl(3-trimethoxysilylpropyl)ammonium chloride (OTC), polyethyleneiminetrimethoxysilane (PEIM), and mixtures thereof.

According to another embodiment of the present invention, the solid support has a pillar structure with pillars extending substantially perpendicularly from the bottom of the cathode chamber.

According to another embodiment of the present invention, the ion exchange membrane conducts electric current and is impermeable for ions and/or gases that are generated by electrolysis in the anode chamber and in the cathode chamber.

According to another embodiment of the present invention, the cathode electrode is formed of a metal, which is able to absorb hydrogen gas.

According to another embodiment of the present invention, anode electrode is formed of a metal, which has a higher standard oxidation potential than that of water and which does not react with water.

According to another embodiment of the present invention, the cathode electrode is formed of palladium (Pd).

According to another embodiment of the present invention, the anode electrode is formed of a metal selected from the group consisting of copper (Cu), lead (Pb), silver (Ag), chromium (Cr), titanium (Ti), nickel (Ni), zinc (Zn), iron (Fe), and tin (Sn).

According to another embodiment of the present invention, the cathode chamber and/or the anode chamber comprise an inlet for introduction of a solution into the chamber and an outlet and for discharge of a solution out of the chamber.

According to another embodiment of the present invention, the microfluidic device for concentrating a sample containing cells or viruses and lysing the cells or viruses may further comprise a first adhesive layer formed on one side of the ion exchange membrane; a second adhesive layer formed on the reverse side of the ion exchange membrane; an anode electrode supporting substrate having a plurality of openings, which are arranged in a ladder-shape, wherein the anode electrode supporting substrate is adhered to the first adhesive layer and; an anode electrode comprising a plurality of openings, which correspond in shape with the openings of the anode electrode supporting substrate, wherein the anode electrode is fixed on the anode electrode supporting substrate with the openings of the anode electrode supporting substrate and the openings of the anode electrode being in alignment; an anode chamber substrate having a cavity forming the anode chamber, wherein the anode chamber substrate is attached to the anode electrode supporting substrate and covers the anode electrode; a cathode chamber substrate comprising a cavity, which forms the cathode chamber, wherein the upper surface of solid support is adhered to the second adhesive layer; and a cathode electrode, which is fixed on the bottom of the cathode chamber.

According to another embodiment of the present invention, the solid support is formed from the cathode chamber substrate.

According to another embodiment of the present invention, the first adhesive layer and the second adhesive layer may respectively have sufficient adhesive power and may be thin enough to conduct electric current.

According to another embodiment of the present invention, the anode electrode supporting substrates and/or the cathode chamber substrate are formed of printed circuit boards (PCBs).

According to another aspect of the present invention, there is provided a method of producing the microfluidic device for concentration of a sample containing cells or viruses and lysis of the cells or viruses, the method comprising producing an anode electrode supporting substrate having a plurality of openings, which are arranged in a ladder-shape, and an anode electrode comprising a plurality of openings, which correspond in shape with the openings of the anode electrode supporting substrate and which is fixed on one side of the anode electrode supporting substrate with the openings of the anode electrode supporting substrate and the openings of the anode electrode being in alignment; fixing an anode chamber substrate having a cavity forming the anode chamber to the anode electrode supporting substrate in order to cover the anode electrode and to form an anode chamber; producing a cathode chamber substrate comprising a cavity, which forms the cathode chamber, and the solid support, and a cathode electrode, which is fixed on the bottom of the cathode chamber; forming a first adhesive layer and a second adhesive layer on the respective sides of an ion exchange membrane; and adhering the anode electrode supporting substrate to the first adhesive layer, and adhering the cathode chamber substrate to the second adhesive layer.

According to an embodiment of the present invention, the production of the anode electrode supporting substrate comprises coating a photoresist film on a metal-coated printed circuit board (PCB); irradiating UV light through a mask having a pattern designed to form the plurality of openings of the anode electrode supporting substrate and the anode electrode, and developing the pattern on the anode electrode thereby forming the plurality of openings of the anode electrode; etching the metal of the exposed metal-coated PCB; and cutting the exposed PCB of the metal-coated PCB to form the openings of the anode electrode supporting substrate.

According to an embodiment of the present invention, the production of the cathode chamber substrate comprises coating a photoresist film on the cathode chamber substrate; irradiating UV light through a mask having a pattern designed to form the solid substrate, and developing the pattern on the cathode chamber substrate; etching the exposed cathode chamber substrate thereby forming the solid substrate and the bottom of the cathode chamber; vapor depositing at least one metal on the photoresist on the upper faces of the solid substrate and on the bottom of the cathode chamber by vacuum evaporation involving heat with an electron beam; removing the photoresist film from the upper faces of the solid substrate; and electroplating the metal-deposited bottom of the cathode chamber substrate with another metal.

According to another embodiment of the present invention, the production of the cathode chamber substrate may further comprise coating the surface of the solid support with a cell-binding substance.

According to another embodiment of the present invention, the formation of the first and second adhesive layers comprises spin coating an adhesive on the respective sides of the ion exchange membrane; adhering one side of the ion exchange membrane, which side is coated with the first adhesive layer, to the anode supporting substrate while spreading the ion exchange membrane using a roller; and bonding the other side of the ion exchange membrane, which side is coated with the second adhesive layer, to the cathode chamber substrate.

According to another embodiment of the present invention, the adhesive may be liquid at ambient temperature and may attain adhesiveness while being cured, when heat or ultraviolet light is applied.

According to another embodiment of the present invention, the adhesion of the first and the second adhesive layers to the anode electrode supporting substrate and the cathode chamber substrate, respectively, is performed by arranging the anode electrode supporting substrate, the ion exchange membrane and the cathode chamber substrate in an array, and applying heat and pressure to the array.

According to another aspect of the present invention, there is provided a method of concentrating a sample containing cells or viruses and lysing the cells or viruses using the microfluidic device, the method comprising introducing a solution containing ions having a standard oxidation potential higher or lower than that of water into the anode chamber of the microfluidic device; introducing a solution containing cells or viruses and ions having a lower standard reduction potential than that of water into the cathode chamber of the microfluidic device; and applying electric current through an anode electrode and an cathode electrode thereby inducing electrolysis in the anode chamber and the cathode chamber and adjusting the pH of the solution in the anode chamber and/or the cathode chamber.

According to another embodiment of the present invention, the ions that are introduced into the anode chamber and that have a lower standard oxidation potential lower than that of water include at least one ion selected from the group consisting of NO₃⁻, F-, SO₄²⁻, PO₄³⁻ and CO₃²⁻.

According to another embodiment of the present invention, the ions that are introduced into the anode chamber and have a standard oxidation potential higher than that of water include Cl⁻.

According to another embodiment of the present invention, the ions that are introduced into the cathode chamber and that have a lower standard oxidation potential lower than that of water include at least one selected from the group consisting of Na⁺, K⁺, Ca²⁺, Mg²⁺ and Al³⁺.

According to another embodiment of the present invention, the pH of the solution in the anode chamber and the cathode chamber may be adjusted by varying the direction of the applied electric current, the intensity of the applied electric current, the duration of electric current application, the width of the electrode, or the thickness of the ion exchange membrane.

### BRIEF DESCRIPTION OF THE DRAWINGS

The above and other features and advantages of the present invention will become more apparent by describing in detail exemplary embodiments thereof with reference to the attached drawings in which:
FIG. 1 illustrates a lateral cross-sectional view of a microfluidic device according to an embodiment of the present invention;
FIG. 2 illustrates a perspective exploded view of a microfluidic device according to an embodiment of the present invention;
FIG. 3A is a diagram illustrating the phenomenon that, when the upper faces of pillars in a microfluidic device according to an embodiment of the present invention are not completely bonded to an ion exchange membrane, a flowing solution flows over the upper faces of the pillars;
FIG. 3B is a diagram illustrating a phenomenon that, when the upper faces of the pillars are completely bonded to an ion exchange membrane in a microfluidic device according to an embodiment of the present invention, the flowing solution flows along the lateral sides of pillars;
FIG. 4 is a flow diagram illustrating the respective steps of a method of producing a microfluidic device according to an embodiment of the present invention;
FIG. 5 is a diagram illustrating an exemplary process of producing an anode electrode supporting substrate of a microfluidic device according to an embodiment of the present invention;
FIG. 6 is a diagram illustrating an exemplary process of producing a cathode chamber substrate of a microfluidic device according to an embodiment of the present invention;
FIG. 7A to FIG. 7C are diagrams respectively illustrating differently shaped upper sides of the cathode chamber substrates produced in the Examples of the present invention; and
FIG. 8 is a photographic image of an electrophoresis gel illustrating the results confirming that microfluidic device according to an embodiment of the present invention can be used for DNA extraction.

### DETAILED DESCRIPTION OF THE INVENTION

The present invention will now be described in more detail with reference to the accompanying drawings, in which exemplary embodiments of the invention are shown. The invention may, however, be embodied in many different forms and should not be construed as being limited to the embodiments set forth herein. Rather, these embodiments are provided so that this disclosure will be thorough and complete, and will fully convey the concept of the invention to those skilled in the art.

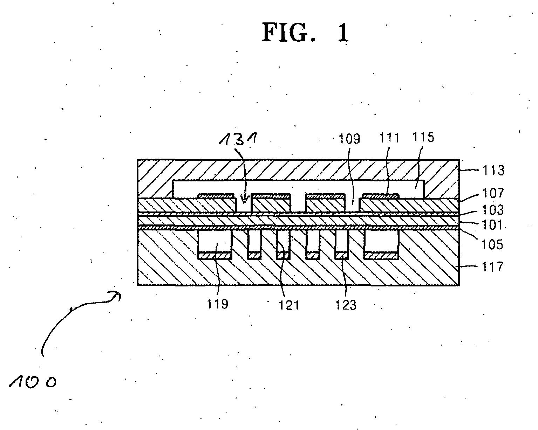

FIG. 1 is a lateral cross-sectional view of a microfluidic device according to an embodiment of the present invention.

Referring to FIG. 1, the microfluidic device 100 according to the current embodiment of the present invention comprises an ion exchange membrane 101, a first adhesive layer 103, a second adhesive layer 105, an anode electrode supporting substrate 107, an anode electrode 111, an anode chamber substrate 113, a cathode chamber substrate 117, and a cathode electrode 123.

The ion exchange membrane 101 is characterized in that the ion exchange membrane 101 conducts electric current but does not allow permeation of ions or gases generated when performing electrolysis in an anode chamber 115 and a cathode chamber 119. For example, the ion exchange membrane 101 may be characterized in that the ion exchange membrane 101 conducts electric current but does not allow permeation of protons and/or hydroxide ions produced upon electrophoresis in the anode chamber 115 and/or the cathode chamber 119. The anode chamber 115 and the cathode chamber 119 are chambers in which a solution can be placed and that comprising an anode electrode 111 and a cathode electrode 123, respectively.

The ion exchange membrane 101 may be a cation exchange membrane or an anion exchange membrane. The cation exchange membrane is a membrane that allows cations to permeate through but shows an approximate 100% resistance to the permeation of anions. On the other hand, the anion exchange membrane is a membrane that allows anions to permeate through but shows an approximate 100% resistance to the permeation of cations.

For instance, the cation exchange membrane may be a strong acid exchange membrane (containing -SO₃⁻ ; Nafion™), or a weak acid exchange membrane (containing -COO⁻), while the anion exchange membrane may be a strong base exchange membrane (containing N⁺(CH₃)) or a weak base exchange membrane (containing N(CH₃)₂).

The cation exchange membrane and the anion exchange membrane are well known in the related art, and one having ordinary skill in the art will be able to select and purchase the appropriate ion exchange membrane 101. For example, the ion exchange membrane 101 is commercially available under the trade names of Nafion™ (Dupont Corp.), Dowex™ (Sigma-Aldrich Co.), and Diaion™ (Sigma-Aldrich Co.).

The first adhesive layer 103 is formed on one side of the ion exchange membrane 101, and the second adhesive layer 105 is formed on the reverse side of the ion exchange membrane 101. It is desirable that the first adhesive layer 103 and the second adhesive layer 105 have sufficient adhesive power but are thin enough to conduct electric current. The first adhesive layer 103 and the second adhesive layer 105 may each be formed by coating both sides of the ion exchange membrane 101 with an adhesive, which is preferably liquid at ambient temperature and attains adhesiveness while being cured when heat is applied, and then heating the adhesive layers. In the current embodiment of the present invention, Dow Corning Primer 1205 was used as the adhesive.

The anode electrode supporting substrate 107 is directly attached to the first adhesive layer 103. The anode electrode supporting substrate 107 comprises openings 109 arranged in a ladder-shape, i.e. a plurality of slots 109, which are aligned parallel to each other like the rungs of a ladder, form a multiplicity of passages through the anode electrode supporting substrate 107. The anode electrode supporting substrate 107 may be formed of a substance selected from the group consisting of a printed circuit board (PCB), silicon wafer, glass, quartz, metals and plastics. For example, the anode electrode supporting substrate 107 may be formed of a PCB.

In the case of using the PCB as the anode electrode supporting substrate 107, the processing of the openings 109 is facilitated, and the width of the slit-shaped openings 109, through which electric current is conducted, can decrease, the number of openings 109 in a single row can be increased, which in turn decreases the electrical resistance. The price of mass production of the microfluidic device can decrease. For instance, the method of forming openings 109 on a PCB can be achieved at a price that is about 100 times cheaper than the method of forming such openings 109 on a glass substrate by sand blasting.

The anode electrode 111 is fixed on the anode electrode supporting substrate 107. The anode electrode 111 is attached to the side of the anode electrode supporting substrate 107 facing away from the reverse side on top of which reverse side the first adhesive layer 103 is deposited on. The anode electrode 111 is shaped in accordance with the openings 109 arranged in ladder-shape, i.e. the anode electrode 111 also comprises a plurality of opening arranged in ladder-shape. Thus, the anode electrode 111 is substantially congruent with the area of the anode electrode supporting substrate 107 comprising the apertures 109, and the anode electrode 111 is placed on top of the anode electrode supporting substrate 107 such that the opening of the anode electrode 111 and the openings of the anode electrode supporting substrate 107 are in alignment leaving the passageways open. According to this embodiment, the number of electrodes in a single transverse row can be increased. Thus, the pH change distribution can be uniformly controlled, and the effects of a pillar-structured chip can be maximized.

The anode electrode 111 may be formed of a metal that has a higher standard oxidation potential than that of water and does not react with, i.e. is chemically inert towards water. In this case, gases are not generated in an anode chamber 115, and thus, the anode chamber 115 does not need to have a gas outlet.

The anode electrode 111 may be formed of a metal selected from all metals that have higher oxidation potentials than that of water and do not react with water, but the anode electrode 111 is not limited thereto.

In general, in terms of the electrolysis of water, oxygen gas and protons are produced in the vicinity of the anode electrode 111 generating foams and lowering the pH of the solution, respectively. To avoid generation of foam, the microfluidic device according to an embodiment of the present invention employs a metal having a higher standard oxidation potential than that of water, so that the metal is oxidized and ionized, without water being electrolyzed, and no gas is generated. Furthermore, a small amount of oxygen that may be generated under certain conditions such as an increase in the voltage or a change in the solute, reacts with the metal having a higher standard oxidation potential than that of water, to binding the oxygen by forming a metal oxide at the anode electrode 111, and thereby, foaming due to generation of oxygen gas does not occur.

A metal that has a higher standard oxidation potential than that of water but reacts with water is not suitable for being used as the anode electrode 111. For example, potassium (K), calcium (Ca), sodium (Na) and magnesium (Mg) cannot be used as the anode electrode 111.

Furthermore, a metal that has a higher standard oxidation potential than that of water but forms an oxidized film too quickly, thereby causing the resistance to increase, is also not suitable for being used as the anode electrode 111. For example, aluminum (AI) is rapidly oxidized to alumina, and thus may not be suitable to be used as the anode electrode 111.

The anode electrode 111 according to an embodiment of the present invention may be formed of a metal selected from the group consisting of copper (Cu), lead (Pb), silver (Ag), chromium (Cr), titanium (Ti), nickel (Ni), zinc (Zn), iron (Fe) and tin (Sn).

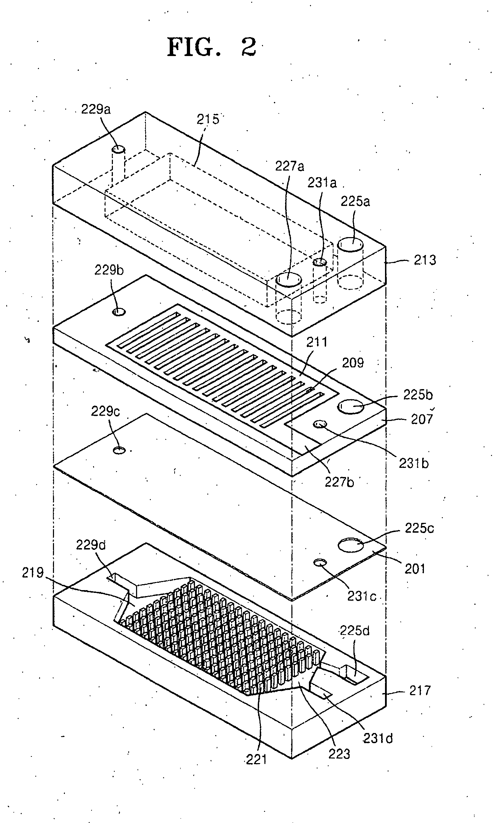

The anode chamber substrate 113 covers the anode electrode 111 thereby forming the anode chamber 115, in which the anode electrode is arranged. In the embodiment shown in Fig. 1 and 2, the anode chamber substrate 113 comprises a cavity 215, which builds the anode chamber 115, when the anode chamber substrate 113 place like a hood on top of that side of anode electrode supporting substrate 107, which supports the anode electrode 111. The cavity of the anode chamber substrate 113 is put over the anode electrode 111 so that the anode electrode 111 is canopied by but not covered up by the anode chamber substrate 113 thus constitutes the side wall and the top of the anode chamber 115. By this, the anode electrode supporting substrate 107 represents the bottom of the anode chamber 115, on which bottom the anode electrode 111 is deposited. The bottom comprises a plurality of slit-shaped openings 119 arranged like the rungs of a ladder, which openings 119 are passageways connecting the anode chamber 115 with in exchange membrane 101.

The cathode chamber substrate 117 is adhered to the second adhesive layer 105 and forms a cathode chamber 119. The cathode chamber substrate 117 comprises a cavity, similar to the cavity of the anode chamber. The cathode chamber 119 comprises a plurality of pillars 121 formed on the bottom of the cavity and extending substantially upright form the bottom of the cavity. The upper faces of the pillars 121 are adhered to the second adhesive layer 105.

According to an embodiment of the present invention, the pillars 121 may have a structure selected from a flat structure, a pillar structure, a bead structure and a sieve structure.

The pillars 121 may be in a state where cells can bind thereto. For instance, the binding of the cells to the pillars 121 can be achieved by means of the physical or chemical properties, such as hydrophobicity or charge, of the surface of the pillars 121. It is desirable that the surface of the pillars 121 is coated with a cell-binding substance. The cell-binding substance is not particularly limited, so long as the cell-binding substance is a substance capable of providing hydrophobicity or charge to the pillars 121 and is capable of capturing cells or viruses. For example, the cell-binding substance may be a hydrophobic substance having a water contact angle of 70° to 90° or a charge donor substance.

A representative method of determining the degree of hydrophobicity of the surface of the pillars 121 comprises the water contact angle. As the water contact angle of the surface of the pillars 121 increases, the degree of hydrophobicity increases. When a solution containing cells or viruses is contacted with a hydrophobic pillars 121 having a water contact angle of 70° to 90°, the cells or viruses bind to the pillars 121 through hydrophobic interaction with the hydrophobic pillars 121, or the like. Hydrophilic pillars 121 hardly bind cells or viruses, as shown in the following Examples. Furthermore, when the water contact angle of the hydrophobic pillars 121 is less or higher than the aforementioned range, the quantity of cells or viruses binding to the hydrophobic pillars 121 decreases (results not shown).

Examples of preferred hydrophobic substance includes octadecyltrichlorosilane (OTS), tridecafluorotetrahydrooctyl trimethoxysilane (DTS), octadecyldimethyl(3-trimethoxysilylpropyl)ammonium chloride (OTC), polyethyleneiminetrimethoxysilane (PEIM), and the like.

The cathode electrode 123 is fixed on the bottom of the cavity of the cathode chamber 119 but not on the pillars 121. The cathode electrode 123 may be formed of a metal that can adsorb hydrogen gas. In this case, since no gas is generated in the cathode chamber 119, thus the cathode chamber 119 does need no gas outlet.

The cathode electrode 123 may be formed of a metal selected from any metal that can absorb hydrogen gas, but the cathode electrode is not particularly limited thereto. For example, the cathode electrode 123 may be formed of palladium (Pd) that is known to have an ability to absorb a large quantity of hydrogen gas (See Bhadra Munasiri, et al., J. Electroanal. Chem., pp 333-337, 1992). In the case of using, for example, Pd for the cathode electrode 123, Pd can prevent the generation of gas by adsorbing to hydrogen gas that is generated in the vicinity of the cathode electrode 123 due to the electrolysis of water. The OH⁻ ions, which are generated simultaneously with the electrolysis of water, raise the pH of the solution in the vicinity of the cathode electrode 123.

FIG. 2 is a perspective view of a microfluidic device according to an embodiment of the present invention shown in an exploded drawing.

Referring to FIG. 2, the microfluidic device comprises, as the main elements, an anode chamber substrate 213, an anode electrode supporting substrate 207, an ion exchange membrane 201 and a cathode chamber substrate 217.

The anode chamber substrate 213 comprises a cavity forming the anode chamber 215, an inlet 229a, an outlet 231 a, a cathode electrode hole 225a, and an anode electrode hole 227a.

The anode electrode supporting substrate 207 comprises a plurality of openings 209 arranged in a ladder-shape, an anode electrode 211 corresponding in shape with the openings 209 of the anode electrode supporting substrate 207 and deposited on to of said anode electrode supporting substrate 207 such that the respective openings of the supporting substrate 209 and the anode electrode 211 are in alignment forming a passageway, an inlet 229b, an outlet 231 b, a cathode electrode hole 225b, and an anode electrode pad 227b.

The ion exchange membrane 201 comprises an inlet 229c, an outlet 231 c, and a cathode electrode hole 225c.

The cathode chamber substrate 217 comprises a cavity forming the cathode chamber 219, a plurality of pillars 221 extending substantially perpendicularly form the bottom the cathode chamber 219, a cathode electrode 223 fixed on the bottom of the cathode chamber 219, an inlet 229d, an outlet 231d, and a cathode electrode pad 225d.

The inlets 229a, 229b, 229c and 229d are aligned together, and the outlets 231 a, 231 b, 231 c and 231 d are also aligned together, wherein the inlet 229d and the outlet 231 d of the cathode chamber substrate are formed as channels, which meet in the cathode chamber 219. Furthermore, the cathode electrode holes 225a, 225b and 225c, and the cathode electrode pad 225d are also aligned together so that a plug can be inserted in said holes for electrically connecting the cathode electrode 123 via the cathode electrode pad 225d with a power supply. Likewise, the anode electrode hole 227a and the anode electrode pad 227b are also aligned together so that another plug can be inserted for electrically connecting the anode electrode 111 via the anode electrode pad 227b with the power supply.

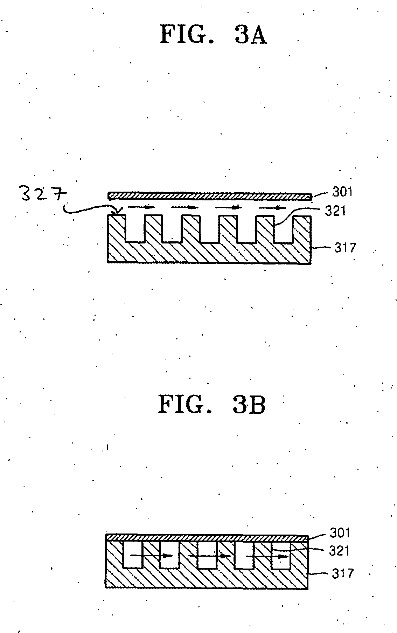

FIG. 3A is a diagram illustrating the phenomenon that, when the upper faces of the pillars 321 in a microfluidic device according to an embodiment of the present invention are not completely bonded to the ion exchange membrane 301, a flowing solution flows over, i.e. on top of the upper faces of the pillars 321 without entering the space between the pillar 321. Referring to FIG. 3A, a solution containing cells or viruses completely flows over the upper faces of the pillars 321, that is, the cells or viruses cannot be captured at the lateral surfaces of the pillars 321, and thus, the concentration of the cells or viruses in the chamber cannot be achieved.

FIG. 3B is a diagram illustrating the phenomenon that, when the upper faces of pillars 321 in a microfluidic device according to an embodiment of the present invention are completely bonded to the ion exchange membrane 301, a flowing solution flows along the lateral sides of the pillars 321 within the chamber. Referring to FIG. 3B, unlike the case illustrated in FIG. 3A, when a solution containing cells or viruses flows between the pillars 321, the cells or viruses can be captured at the pillars 321, and thus, the cells or viruses can be concentrated. The microfluidic device according to embodiments of the present invention solves the problem shown in Fig. 3A by introducing a first adhesive layer and a second adhesive layer used as a bonding means between the ion exchange membrane and the substrates.

In the microfluidic device according to an embodiment of the present invention, the anode chamber and the cathode chamber refer to spaces that can hold a substance such as a fluid, and may be microchambers which can hold a substance up to a volume of sub-microliters, but the microchambers are not limited thereto.

The microfluidic device according to an embodiment of the present invention is not particularly limited in the specific form, structure, size and the like.

The cathode chamber and the anode chamber of the microfluidic device according to an embodiment of the present invention may respectively further comprise an inlet and/or outlet for introduction and discharge of a solution, and may further comprise a micropump for receiving and discharging of the solution.

In the microfluidic device according to an embodiment of the present invention, a solution containing ions, which have a standard oxidation potential higher or lower than that of water, namely, an electrolyte to be electrolyzed can be introduced into the anode chamber. The ions having a lower standard oxidation potential than that of water may include at least one type of ion including anions such as NO₃⁻ , F⁻, SO₄²⁻, PO₄³⁻ and CO₃²⁻, and the ions having a higher standard oxidation potential than that of water may be provided by an electrolyte containing Cl⁻ ions, but the present invention is not limited to thereto.

If the solution introduced into the anode chamber contains a compound having a lower standard oxidation potential than that of water and when electrolysis is performed using the microfluidic device according to an embodiment of the present invention, water is electrolyzed in the anode chamber generating oxygen gas and H⁺ ions. In this case, the pH of the solution in the anode chamber decreases due to the presence of the H⁺ ions. On the other hand, as described above, when a metal, which has a higher standard oxidation potential than that of water and which does not react with water, is used as the anode electrode, the metal having a higher standard oxidation potential than that of water is oxidized thereby consuming oxygen, and hence oxygen gas is not generated. The Cl⁻ ions having a higher standard oxidation potential than that of water can be particularly used only for the purpose of cell lysis.

In the microfluidic device according to embodiments of the present invention, a solution containing cells or viruses and kinds of ions having a lower standard oxidation potential than that of water can be introduced into the cathode chamber. Examples of the ions include Na⁺, K⁺, Ca²⁺, Mg²⁺, Al³⁺ and the like, but are not limited thereto. Therefore, in the case of performing electrolysis using a microfluidic device according to an embodiment of the present invention, water is electrolyzed in the cathode chamber generating hydrogen gas and OH⁻ ions. In this case, the solution in the cathode chamber attains a higher pH due to the presence of the OH⁻ ions.

Meanwhile, as described in the above, when a metal, which can adsorb hydrogen gas, is used as the cathode electrode, the hydrogen gas thus generated is adsorbed, and no gas bubbles are generated.

According to an embodiment of the present invention, the cells or viruses may include bacterial cells, bacteriophages, plant cells, animal cells, plant viruses, animal viruses and the like, but the specific type of cells or viruses is not particularly limited thereto.

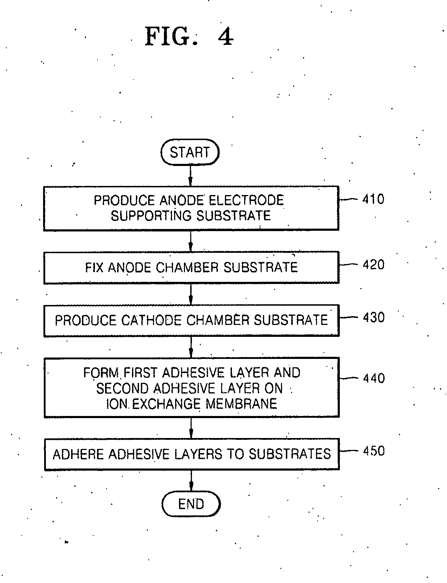

FIG. 4 is a flow diagram illustrating the respective steps of a method of producing a microfluidic device according to an embodiment of the present invention.

Referring to FIG. 4, an anode electrode supporting substrate is produced in a first step 410. The anode electrode supporting substrate 107 of step 410 comprises a ladder-shaped anode electrode 111, which is attached on one surface of the anode electrode supporting substrate 107. Both the anode electrode supporting substrate 107 and the anode electrode 111 include a openings 109 that are arranged like the rung of a ladder, whereby the openings of the anode electrode supporting substrate 107 and the openings of the anode electrode 111 in shape correspond to each other and are arranged in alignment with each other.

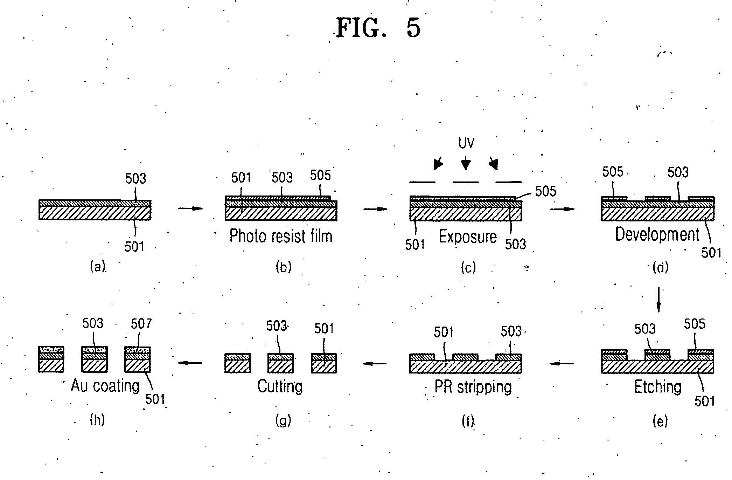

FIG. 5 is a schematic diagram illustrating an example of the process step 410 of producing an anode electrode supporting substrate of a microfluidic device according to an embodiment of the present invention.

Referring to FIG. 5, in order to produce the anode electrode supporting substrate, firstly a PCB 501 is coated with a metal 503 (step (a)) in advance to coating the metal 503 with a photoresist film 505 (step (b)); UV light is irradiated through a mask having a pattern designed to form a ladder-shaped anode electrode thereby exposing only the predetermined regions the photoresist film 505 to the UV light (step (c)); and the pattern is developed, i.e. the regions of the photoresist 505 that were irradiated with UV light are removed (step (d)). Subsequently, the regions of exposed metal 503, where the photoresist was removed in step (d), are etched away (step (e)), and the photoresist film 505 is peeled off the non-exposed remaining metal 503(step (f)). Then, the exposed PCB 501, where the metal 503 was removed in step (e), is etched to form openings (step (g)); and another metal 507 is coated on the remaining metal 503 (step (h)).

Referring to step 420 of FIG. 4, subsequent to producing the anode electrode supporting substrate 107 comprising the anode electrode 111, the anode chamber substrate 113 is fixed on the anode electrode supporting substrate, whereby the anode chamber substrate 113 covers the anode electrode, and thereby an anode chamber is formed, with the anode electrode 111 arranged inside said anode electrode chamber.

Next, a cathode chamber substrate 117 forming a cathode chamber is produced, which includes a solid support, for example a plurality of pillars, and a cathode electrode that is fixed within the cathode chamber (430).

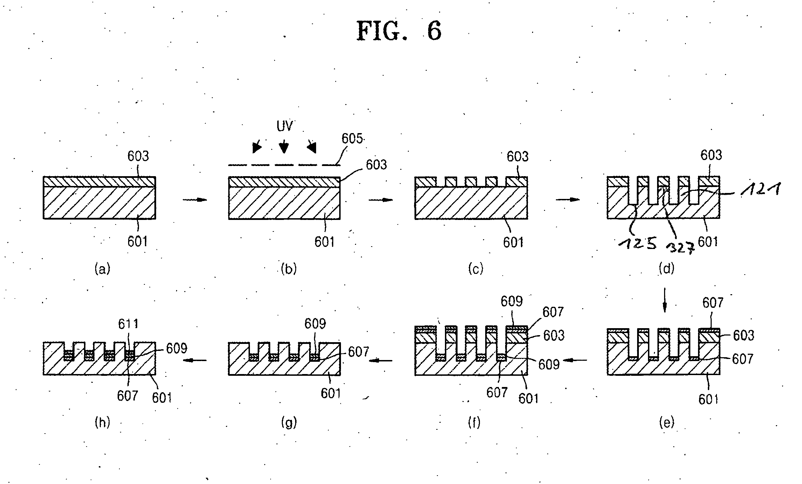

FIG. 6 is a schematic diagram illustrating the process step 430 of producing a cathode chamber substrate 117 of a microfluidic device 100 according to an embodiment of the present invention.

Referring to FIG. 6, in order to produce the cathode chamber substrate, a photoresist film 603 is first coated on a silicon wafer 601 (step (a)), UV light is irradiated through a mask 605 having a pattern designed to form pillars, i.e. the pattern of the mask corresponds to the pattern of the upper surfaces of the pillars to be formed. The pattern is developed, thereby removing the UV-irradiated areas of the photoresist film 603 (step (c)). Subsequently, the exposed silicon wafer 601, where the photoresist 603 was removed in step (c), is etched forming the plurality of pillars (step (d)), and two metals 607 and 609 are sequentially vapor deposited on the bottom of the silicon wafer 601 and on top of the photoresist 603 covering the top surfaces of the pillars by vacuum evaporation involving heat with an electron beam (steps (e) and (f)). Then, the photoresist film 603 is removed using a solvent (at this time, the metals 607 and 609 deposited on the photoresist film 603 is removed as well) (step (g)), and another metal 611 is electroplated on top of the metal 609 deposited on the bottom of the silicon wafer 601 (step (h)). The production of the cathode chamber substrate may further comprise coating a cell-binding substance on the surfaces of the pillars formed.

Referring to FIG. 4 again, a first adhesive layer 103 and a second adhesive layer 105 are formed on the respective sides of an ion exchange membrane 101 in the next step 440.

The formation of the first 103 and second adhesive layers 105 may comprise spin coating an adhesive on both sides of the ion exchange membrane 101. This is followed by adhering one side of the ion exchange membrane 101, said side being coated with the first adhesive layer 103, to the side of the anode electrode supporting substrate that is opposite attached anode electrode 111, while spreading the ion exchange membrane with a roller. This is followed by bonding the reverse side of the ion exchange membrane 101, said reverse side being coated with the second adhesive layer 105, to the cathode chamber substrate so that the upper surfaces of the pillars are bound to the ion exchange membrane 101 via the second adhesive layer 105.

The adhesive may be liquid at ambient temperature and may attain adhesiveness while being cured, when heat or ultraviolet light is applied. In the embodiments of the present invention, Dow Corning Primer 1205 was used as the adhesive.

Subsequently, the anode electrode supporting substrate 107 adheres to the first adhesive layer 103, and the cathode chamber substrate 117 adheres to the second adhesive layer 115 in step 450. The adhesion of the first 103 and second adhesive layers 105 and the anode electrode supporting and cathode chamber substrates can be performed by arranging the anode electrode supporting substrate 107, the ion exchange membrane 101 and the cathode chamber substrate 117 in an array as described above, and thereto applying heat and pressure for curing the adhesive and thereby fixing the components of the microfluidic device 100 to each other.

In addition, the present invention provides a method of concentrating and lysing cells or viruses and the cells or viruses using a microfluidic device according to an embodiment of the present invention. The method comprises the step of introducing a solution, which contains ions having a standard oxidation potential higher or lower than that of water into the anode chamber 115 of the microfluidic device 100; introducing a solution containing cells or viruses and ions having a lower standard reduction potential than that of water into the cathode chamber 119 of the microfluidic device 100; and applying an electric current through the anode electrode 111 and the cathode electrode 123 thereby inducing electrolysis in the anode chamber 115 and the cathode chamber 119, and adjusting the pH of the solution introduced into the anode chamber or the cathode chamber by controlling the electrolysis.

Examples of an anion having a lower standard oxidation potential than that of water, an anion having a higher standard oxidation potential than that of water, and a cation having a lower standard reduction potential than that of water, and examples of cells or viruses are as described above. However, steps (a) and (b) may be performed simultaneously or sequentially.

The pH of the solutions can be adjusted by varying the direction of the applied electric current, the intensity of the applied electric current, the duration of the application of electric current, the width of the electrode, or the thickness of the ion exchange membrane. Precise values of the direction of the applied electric current, the intensity of the applied electric current, the duration of the applied electric current, the width of the electrode, and the thickness of the ion exchange membrane may vary according to the desired pH, the volume of the chamber, or the like, and one ordinarily skilled in the art is able to determine these parameters easily by experimentation.

When a sample solution containing NaCl, which is most abundantly contained in biological sample solutions, is introduced into the anode chamber and the cathode chamber and an electrophoresis is performed, not water but the chloride ions are electrolyzed and oxidized in the anode chamber generating chlorine gas. Thus, a smaller amount of protons is generated in the anode chamber than the amount of hydroxide ions that are generated in the cathode chamber. The amount of protons in the anode chamber in this case results from a reaction of chlorine gas with water, and varies according to the conditions for dissolving of the chlorine gas, thus making it difficult to control the pH of the sample solution. In order to solve such problems, the present invention uses a compound having a standard oxidation potential lower than that of water and a compound having a standard reduction potential lower than that of water in the anode chamber and the cathode chamber, respectively. However, in the case of only lysing cells, a sample solution containing NaCl can be introduced to the anode electrode and the cathode electrode, and then electrolyzed, so that the cells can be lysed at the cathode electrode.

According to an embodiment of the present invention, a solution, which contains a compound having a lower standard reduction potential than that of water, is introduced into the cathode chamber. Due to the reduction potential of said compound, water is electrolyzed and reduced in the cathode chamber generating hydrogen gas and OH⁻ ions in the cathode chamber. Moreover, since a solution, which contains a compound having a lower standard reduction potential than that of water, is introduced into the anode chamber, water is electrolyzed and oxidized in the anode chamber thereby generating oxygen gas and H⁺ ions in the anode chamber. As a result, the solution in the cathode chamber is basic in terms of pH, while the solution in the anode chamber is acidic in terms of pH.

Further, as discussed above, when a metal having a higher standard oxidation potential than that of water and which does not react with water is used as the anode electrode and a metal that can adsorb hydrogen gas is used as the cathode electrode, gas formation in the anode chamber and the cathode chamber can be prevented.

Hereinafter, the present invention will be described in detail with reference to the following Examples. However, these Examples are for illustrative purposes only, and the scope of the present invention is not intended to be limited by the Examples.

### EXAMPLE 1

### Production of the microfluidic device

An anode chamber substrate 213 as illustrated in FIG. 2 was produced using a silicon wafer.

An anode electrode supporting substrate 207 was produced using the method illustrated in FIG. 5. A PCB was used as the anode electrode supporting substrate 207, and lead was coated thereon forming the anode electrode 211. The breadth and length of the anode electrode supporting substrate 207 were 14 mm and 34 mm, respectively, the width of the openings was 1.0 mm, and the width of a transverse electrode unit was 0.5 mm.

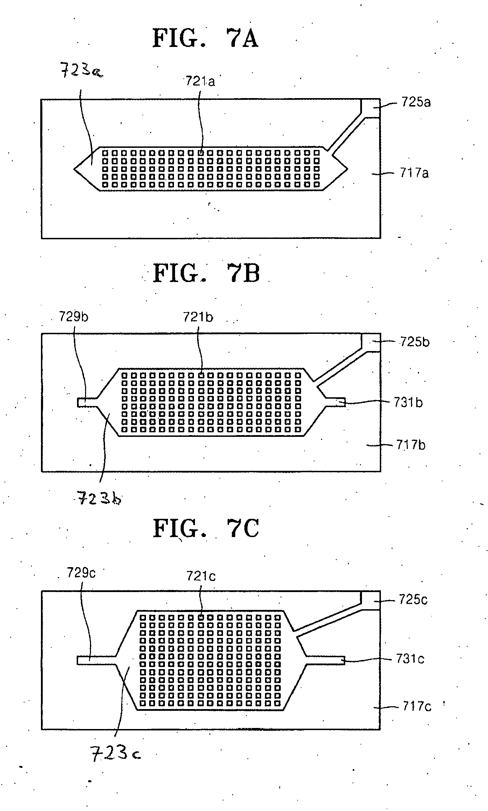

A cathode chamber substrate 217 was produced using the method illustrated in FIG. 6. The silicon wafer was used as the substrate, and palladium was coated thereon forming the cathode electrode 223. FIG. 7A is a diagram illustrating the upper surface of a cathode chamber substrate 717a produced in an Example of the present invention. Referring to FIG. 7A, a plurality of pillars 721 a is formed on the cathode chamber substrate 717a. A cathode electrode 723a is formed on the bottom of the cathode chamber and, in addition to the pillars 721 a, a cathode electrode pad 725a is provided for the connection of the cathode electrode 723a to a power supply. The cathode electrode pad 725a is arranged remote from the cathode chamber in one corner of the cathode chamber substrate 717a, which cathode electrode pad 725a is electrically connected to the cathode electrode 723a via a connection line.

Dow Corning Primer 1205 was spin coated (500 rpm, 5 s; 700 rpm, 10 s) on the silicon wafer, namely the side comprising the upper faces of the pillars as an adhesive forming the second adhesive layer, a cation exchange membrane containing -SO₃⁻-Na⁺ group is bonded on the silicon wafer coated with the first adhesive layer using a roller, and then Dow Corning Primer 1205 was spin coated on the exposed side of the cation exchange membrane (500 rpm, 5 s; 1500 rpm, 10 s) to form the first adhesive layer on the cation exchange membrane. The Dow Corning Primer 1205 is characterized in that the Dow Corning Primer 1205 has a low viscosity, comparable to the viscosity of water at room temperature, and has no adhesiveness in this state. But when heat is applied to the Dow Corning Primer 1205, the adhesive is solidified and obtains adhesiveness.

The anode chamber substrate, the anode electrode supporting substrate, the cation exchange membrane, and the cathode chamber substrate produced as described above were arranged in an array, and were bonded together by heating the array to a temperature of 120°C and applying a pressure of 1 ton for 30 minutes in order to produce the microfluidic device of the present invention. The volumes of the produced cathode chamber and anode chamber were respectively 10 µl.

### EXAMPLES 2 to 6

### Production of the microfluidic device

Microfluidic devices of Examples 2 through 6 were produced in the same manner as described in Example 1, except that the shape of the cathode chamber substrate differed.

FIG. 7B and FIG. 7C are diagrams illustrating the upper sides of the cathode chamber substrates of different shapes that were produced in Example 2 and Example 3, respectively.

### COMPARATIVE EXAMPLE 1

### Production of the microfluidic device

A microfluidic device was produced in the same manner as described in Example 1, except that the first adhesive layer and the second adhesive layer were not provided therein.

### EXPERIMENTAL EXAMPLE 1

### Confirmation of the effects of a microfluidic device on cell concentration out of a sample containing cells

The effects of capturing and concentrating cells from a sample containing cells using the microfluidic devices produced in Examples 1 through 6 and Comparative Example 1 were confirmed.

A 500 µl solution containing *Escherichia coli* cells at a cell concentration of 2.5x10⁶ cells/ml flowed through the cathode chamber of each of the microfluidic devices at a flow rate of 30 µl/min, and then the number of cells captured in the cathode chamber was counted to determine a cell capture ratio.

The results are presented in Table 1. As shown in Table 1, the cell capture ratios obtained using the microfluidic devices produced in the Examples 1 to 6 of the present invention were higher than 90%. It was found in the Comparative Example 1 that no cells were captured, when cells using a microfluidic device wherein the upper faces of the pillars of the microfluidic device are only mechanically adjoined to the ion exchange membrane, i.e. without bonding the upper faces of the pillars with an adhesive.

**[Table 1]**

| Example 1 | Example 2 | Example 3 | Example 4 | Example 5 | Example 6 | Average | Standard deviation | Comp. Ex.1 |
|---|---|---|---|---|---|---|---|---|
| 89.61 % | 89.24 % | 90.53 % | 90.45 % | 90.49 % | 90.23 % | 90.1 % | 0.5 | App. 0 % |

### EXPERIMENTAL EXAMPLE 2

### Confirmation of the effects of using a microfluidic device for DNA extraction

The effects of using the microfluidic devices produced in Examples 1 through 3 and Comparative Example 1 for DNA extraction were examined.

A 500 µl sample solution containing *Escherichia coli* cells flowed through the cathode chamber of each of the microfluidic devices at a flow rate of 300 µl/min. Subsequently, 10 µl of a 55 mM solution of Na₂SO₄ was introduced into the anode chamber, and then an electric current of 2 mA was applied through the cathode electrode and the anode electrode for 30 seconds. After completing electrolysis as described above, 20 µl of a 55 mM solution of Na₂SO₄ was introduced into the cathode chamber in order to elute from the cathode chamber the solution that resided during the electrophoresis in the cathode chamber. Subsequently, the recovered eluted solution was used to perform a PCR reaction and electrophoresis under the same conditions. The DNA concentration was measured using a Lap Chip.

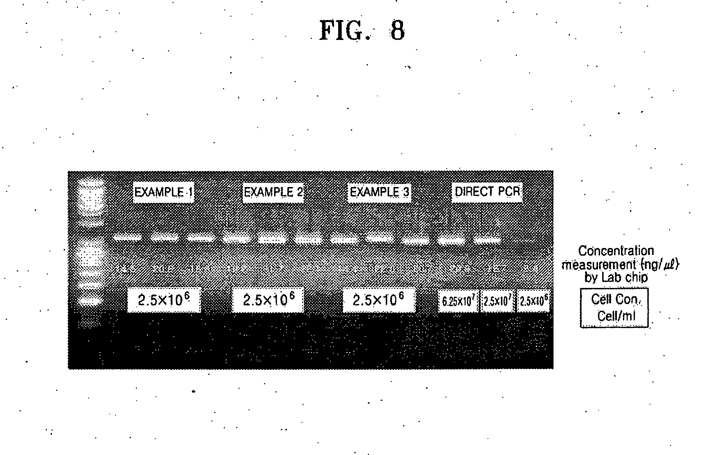

FIG. 8 is a photographic image of the electrophoresis gel obtained according to Experimental Example 2, the image showing the results that confirm the effect of extracting DNA using the microfluidic device according to an embodiment of the present invention on DNA extraction.

FIG. 8 shows the result of repeating a PCR reaction three times using *E. coli* cells at an initial cell concentration of 2.5×10⁶ cells/ml in the sample solution when using the microfluidic device of Example 1. The concentrations of DNA obtained with the device of Example 1 were 14.5 ng/µl, 20.6 ng/µl and 15.6 ng/µl. Furthermore, the concentrations of DNA obtained in the same manner using the microfluidic device of Example 2 were 19.2 ng/µl, 31.7 ng/µl and 22.1 ng/µl, and the concentrations of DNA obtained in the same manner using the microfluidic device of Example 3 were 14.4 ng/µl, 22.0 ng/µl and 20.7 ng/µl.

The term "direct PCR" in Fig. 8 refers to the results obtained by immediately performing a PCR reaction, i.e. without introducing the sample solution containing *E. coli* cells into the microfluidic device. The concentration of DNA obtained with a solution having a cell concentration of 2.5×10⁶ cells/ml was 3.4 ng/µl, the concentration of DNA obtained with a solution at a cell concentration of 2.5×10⁷ cells/ml was 12.7 ng/µl, and the concentration of DNA obtained with a solution at a cell concentration of 6.25×10⁷ cells/ml was 29.9 ng/µl.

From the results, it was found that the average concentration of extracted DNA obtained with solutions at the same concentration, namely, 2.5x10⁶ cells/ml, was 20.09 ng/µl. This was significantly higher in comparison to the DNA concentration of 3.4 ng/µl obtained by performing a direct PCR reaction using a solution having the same cell concentration. Accordingly, it can be seen that the microfluidic device according to embodiments of the present invention can simultaneously and effectively perform cell concentration of a sample containing cells or viruses and the lysis of the cells or viruses.

As discussed above, the microfluidic device of the present invention can effectively perform the cell concentration of a sample containing cells or viruses and the lysis of the cells or viruses in a single chamber. Therefore, the size of a lab-on-a-chip can be reduced by using the microfluidic device and the method of concentrating cells from a sample containing cells or viruses and lysing the cells or viruses using the microfluidic device of the present invention. Furthermore, when the phenomenon of the chamber dividing membrane swelling is prevented, and the change in the chamber volume is decreased, a sample of a predetermined volume can be readily introduced into the chamber. Further, when the anode electrode is formed in a ladder shape that is widely and evenly distributed on the anode electrode supporting substrate, the electric current flows easier due to a reduced resistance of the anode, and the pH adjustments can be performed more efficient.

## Claims

1. A microfluidic device (100) for concentrating cells from a sample containing cells or viruses and for lysing the cells or viruses, the device comprising
an anode chamber (115) containing an anode electrode (111);
a cathode chamber (119) containing a cathode electrode (123); and
an ion exchange membrane (101) separating the anode chamber (115) and the cathode chamber (119),
wherein a solid support (121) capable of retaining cells or viruses from the sample containing cells or viruses is disposed in the cathode chamber (119), wherein the surface of the solid support (121) is coated with a cell-binding substance, and wherein the solid support (121) has a structure selected from the group consisting of a flat structure, a pillar structure, a bead structure and a sieve structure.

2. The microfluidic device (100) of claim 1, wherein the cell-binding substance is a hydrophobic substance having a water contact angle of from 70° to 90°, or a charge donor substance.

3. The microfluidic device (100) of claim 2, wherein the hydrophobic substance is selected from the group consisting of octadecyltrichlorosilane (OTS), tridecafluorotetrahydrooctyl trimethoxysilane (DTS), octadecyldimethyl(3-trimethoxysilylpropyl)ammonium chloride (OTC), polyethyleneiminetrimethoxysilane (PEIM) and mixtures thereof.

4. The microfluidic device (100) of any of claims 1 to 3, wherein the solid support (121) has a pillar structure with pillars (121) extending substantially perpendicularly from the bottom (125) of the cathode chamber (119).

5. The microfluidic device (100) according to any of claims 1 to 4, wherein the ion exchange membrane (101) conducts electric current and is impermeable for ions and/or gases that are generated by electrolysis in the anode chamber (115) and in the cathode chamber (119).

6. The microfluidic device (100) according to any of claims 1 to 5, wherein the cathode electrode (123) is formed of a metal, which is able to absorb hydrogen gas.

7. The microfluidic device (100) according to any of claims 1 to 6, wherein the anode electrode (111) is formed of a metal, which has a higher standard oxidation potential than that of water and which does not react with water.

8. The microfluidic device (100) of claim 7, wherein the cathode electrode (123) is formed of palladium (Pd).

9. The microfluidic device of claim 8, wherein the anode electrode (111) is formed of a metal selected from the group consisting of copper (Cu), lead (Pd), silver (Ag), chromium (Cr), titanium (Ti), nickel (Ni), zinc (Zn), iron (Fe) and tin (Sn).

10. The microfluidic device (100) according to any of claims 1 to 9, wherein the cathode chamber (119) and/or the anode chamber (115) comprise an inlet (229) for introduction of a solution into the chamber (115, 119) and an outlet (231) and for discharge of a solution out of the chamber (115, 119).

11. The microfluidic device (100) according to any of claims 1 to 10, further comprising
a first adhesive layer (103) formed on one side of the ion exchange membrane (101);
a second adhesive layer (105) formed on the reverse side of the ion exchange membrane (101);
an anode electrode supporting substrate (107) having a plurality of openings (109), which are arranged in a ladder-shape, wherein the anode electrode supporting substrate (107) is adhered to the first adhesive layer (103) and;
an anode electrode (111) comprising a plurality of openings (131), which correspond in shape with the openings (109) of the anode electrode supporting substrate (107), wherein the anode electrode (111) is fixed on the anode electrode supporting substrate (107) with the openings (109) of the anode electrode supporting substrate (107) and the openings (131) of the anode electrode (111) being in alignment;
an anode chamber substrate (113) having a cavity forming the anode chamber (115), wherein the anode chamber substrate (113) is attached to the anode electrode supporting substrate (107) and covers the anode electrode (111);
a cathode chamber substrate (117) comprising a cavity, which forms the cathode chamber (119), wherein the upper surface (327) of solid support (121) is adhered to the second adhesive layer (105); and
a cathode electrode (123), which is fixed on the bottom (125) of the cathode chamber (119).

12. The microfluidic device of claim 11, wherein the solid support (121) is formed from the cathode chamber substrate (117).

13. The microfluidic device (100) according to any of claim 11 to 12, wherein the first adhesive layer (103) and the second adhesive layer (105) are able to conduct electric current.

14. The microfluidic device (100) according to any of claims 11 to 13, wherein the anode electrode supporting substrates (107) and/or the cathode chamber substrate (117) are formed of printed circuit boards (PCBs).

15. A method of producing the microfluidic device (100) according to any of claims 1 to 14, the method comprising
producing an anode electrode supporting substrate (107) having a plurality of openings (109), which are arranged in a ladder-shape, and an anode electrode (111) comprising a plurality of openings (131), which correspond in shape with the openings (109) of the anode electrode supporting substrate (107) and which is fixed on one side of the anode electrode supporting substrate (107) with the openings (109) of the anode electrode supporting substrate (107) and the openings (131) of the anode electrode (111) being in alignment;
fixing an anode chamber substrate (113) having a cavity forming the anode chamber (115) to the anode electrode supporting substrate (107) in order to cover the anode electrode (111) and to form an anode chamber (115);
producing a cathode chamber substrate (117) comprising a cavity, which forms the cathode chamber (119), and the solid support (121), and a cathode electrode (123), which is fixed on the bottom of the cathode chamber (119);
forming a first adhesive layer (103) and a second adhesive layer (105) on the respective sides of an ion exchange membrane (101); and
adhering the anode electrode supporting substrate (107) to the first adhesive layer (103), and adhering the cathode chamber substrate (117) to the second adhesive layer (105).

16. The method of claim 15, wherein the producing of the anode electrode supporting substrate (107) comprises
coating a photoresist film (505) on a metal-coated printed circuit board (PCB 501, 503);
irradiating UV light through a mask having a pattern designed to form the plurality of openings (109, 131) of the anode electrode supporting substrate (107) and the anode electrode (111), and developing the pattern on the anode electrode (111) thereby forming the plurality of openings (131) of the anode electrode (111);
etching the metal (503) of the exposed metal-coated PCB (501, 503); and
cutting the exposed PCB (501) of the metal-coated PCB (501, 503) to form the openings (119) of the anode electrode supporting substrate (107).

17. The method of claim 15, wherein the producing of the cathode chamber substrate (117) comprises
coating a photoresist film (503) on the cathode chamber substrate (601);
irradiating UV light through a mask having a pattern designed to form the solid substrate (121), and developing the pattern on the cathode chamber substrate (601);
etching the exposed cathode chamber substrate (601) thereby forming the solid substrate (121) and the bottom (125) of the cathode chamber (119);
vapor depositing at least one metal (607, 609) on the photoresist (603) on the upper faces (327) of the solid substrate (121) and on the bottom (125) of the cathode chamber (119) by vacuum evaporation involving heat with an electron beam;
removing the photoresist film (603) from the upper faces (327) of the solid substrate (121); and
electroplating the metal-deposited (607, 609) bottom (125) of the cathode chamber substrate (117) with another metal (611).

18. The method of claim 17, wherein the producing of the cathode chamber substrate (117) further comprises coating the surface of the solid support (121) with a cell-binding substance.

19. The method of claim 15, wherein the forming of the first (103) and second adhesive layers (105) comprises
spin coating an adhesive on the respective sides of the ion exchange membrane (101);
adhering one side of the ion exchange membrane, which side is coated with the first adhesive layer (103), to the anode supporting substrate (107) while spreading the ion exchange membrane (101) using a roller; and
bonding the other side of the ion exchange membrane, which side is coated with the second adhesive layer (105), to the cathode chamber substrate (117).

20. The method of claim 15, wherein the adhesive is liquid at room temperature and attains adhesiveness while being cured when heat or UV light is applied to the adhesive.

21. The method of claim 15, wherein the adhesion of the first (103) and the second adhesive layers (105) to the anode electrode supporting substrate (107) and the cathode chamber substrate (117), respectively, is performed by arranging the anode electrode supporting substrate (107), the ion exchange membrane (101) and the cathode chamber substrate (117) in an array, and applying heat and pressure to the array.

22. A method of concentrating cells from a sample containing cells or viruses and for lysing the cells or viruses using the microfluidic device (100) according to any of claims 1 to 14, the method comprising
introducing a solution containing ions having a standard oxidation potential higher or lower than that of water into the anode chamber (115) of the microfluidic device (100);
introducing a solution containing cells or viruses and ions having a lower standard reduction potential than that of water into the cathode chamber (119) of the microfluidic device (100); and
applying electric current through an anode electrode (111) and a cathode electrode (123) thereby inducing electrolysis in the anode chamber (115) and the cathode chamber (119) and adjusting the pH of the solution in the anode chamber (115) and/or the cathode chamber (199).

23. The method of claim 22, wherein the ions that are introduced into the anode chamber (115) and that have a lower standard oxidation potential than that of water include at least one ion selected from the group consisting of NO₃⁻, F⁻, SO₄²⁻, PO₄³⁻ and CO₃²⁻.

24. The method of claim 22, wherein the ions that are introduced into the anode chamber (115) and that have a higher standard oxidation potential than that of water include Cl⁻.

25. The method of claim 22, wherein the ions that are introduced into the cathode chamber include at least one ion selected from the group consisting of Na⁺, K⁺, Ca²⁺, Mg²⁺ and Al³⁺.

26. The method of claim 22, wherein the pH of the solutions in the anode chamber (115) and the cathode chamber (119) is adjusted by varying the direction of the applied electric current, the intensity of the applied electric current, the duration of the applied electric current, the width of the electrode, or the thickness of the ion exchange membrane (101).

## Patentansprüche

1. Mikrofluidische Vorrichtung (100) zum Konzentrieren von Zellen aus einer zellen- oder virenhaltigen Probe und zum Lysieren der Zellen oder Viren, wobei die Vorrichtung umfasst:
eine Anodenkammer (115) enthaltend eine Anodenelektrode (111);
eine Kathodenkammer (119) enthaltend eine Kathodenelektrode (123); und
eine Ionenaustauschmembran (101), welche die Anodenkammer (115) und die Kathodenkammer (119) trennt,
wobei ein fester Träger (121), welcher in der Lage ist, Zellen oder Viren aus der zellen-oder virenhaltigen Probe zu halten, in der Kathodenkammer (119) angeordnet ist, wobei die Oberfläche des festen Trägers (121) mit einer zellenbindenden Substanz beschichtet ist und wobei der feste Träger (121) eine Struktur aufweist, gewählt aus der Gruppe bestehend aus einer flachen Struktur, einer Säulenstruktur, einer Kugelstruktur und einer Siebstruktur.

2. Mikrofluidische Vorrichtung (100) nach Anspruch 1, wobei die zellenbindende Substanz eine hydrophobe Substanz ist, mit einem Wasserkontaktwinkel von 70° bis 90° oder einer ladungsabgebenden Substanz.

3. Mikrofluidische Vorrichtung (100) nach Anspruch 2, wobei die hydrophobe Substanz gewählt ist aus der Gruppe bestehend aus Octadecyltrichlorsilan (OTS), Tridecafluortetrahydrooctyltrimethoxysilan (DTS), Octadecyldimethyl(3-trimethoxysilylpropyl)ammoniumchlorid (OTC), Polyethyleneimintrimethoxysilan (PEIM) und deren Mischungen.

4. Mikrofluidische Vorrichtung (100) nach einem der Ansprüche 1 bis 3, wobei der feste Träger (121) eine Säulenstruktur aufweist, mit Säulen (121), welche sich im Wesentlichen senkrecht von dem Boden (125) der Kathodenkammer (119) aus erstrecken.

5. Mikrofluidische Vorrichtung (100) nach einem der Ansprüche 1 bis 4, wobei die lonenaustauschmembran (101) elektrischen Strom leitet und für Ionen und/oder Gase, welche
durch Elektrolyse in der Anodenkammer (115) und in der Kathodenkammer (119) erzeugt werden, undurchdringbar ist.

6. Mikrofluidische Vorrichtung (100) nach einem der Ansprüche 1 bis 5, wobei die Kathodenelektrode (123) aus einem Metall gebildet ist, welches Wasserstoffgas absorbieren kann.

7. Mikrofluidische Vorrichtung (100) nach einem der Ansprüche 1 bis 6, wobei die Anodenelektrode (111) aus einem Metall gebildet ist, welches ein höheres Standardoxidationspotential als das von Wasser aufweist, und welches nicht mit Wasser reagiert.

8. Mikrofluidische Vorrichtung (100) nach Anspruch 7, wobei die Kathodenelektrode (123) aus Palladium (Pd) gebildet ist.

9. Mikrofluidische Vorrichtung (100) nach Anspruch 8, wobei die Anodenelektrode (111) aus einem Metall gebildet ist, gewählt aus der Gruppe bestehend aus Kupfer (Cu), Blei (Pd), Silber (Ag), Chrom (Cr), Titan (Ti), Nickel (Ni), Zink (Zn), Eisen (Fe) und Zinn (Sn).

10. Mikrofluidische Vorrichtung (100) nach einem der Ansprüche 1 bis 9, wobei die Kathodenkammer (119) und/oder die Anodenkammer (115) einen Einlass (229) zur Einführung einer Lösung in die Kammer (115, 119) und einen Auslass (231) zur Entfernung einer Lösung aus der Kammer (115, 119), umfasst.

11. Mikrofluidische Vorrichtung (100) nach einem der Ansprüche 1 bis 10, des Weiteren umfassend
eine erste adhesive Schicht (103), welche auf einer Seite der Ionenaustauschmembran (101) gebildet ist,
eine zweite adhesive Schicht (105), welche auf der Rückseite der lonenaustauschmembran (101) gebildet ist;
ein Anodenelektrodenträgersubstrat (107) mit einer Vielzahl von Öffnungen (109), welche leiterförmig angeordnet sind, wobei das Anodenelektrodenträgersubstrat (107) an der ersten adhesiven Schicht (103) haftet und
eine Anodenelektrode (111) umfassend eine Vielzahl von Öffnungen (131), welche in der Form mit den Öffnungen (109) des Anodenelektrodenträgersubstrats (107) übereinstimmen, wobei die Anodenelektrode (111) auf dem Anodenelektrodenträgersubstrat (107) befestigt ist, wobei die Öffnungen (109) des Anodenelektrodenträgersubstrats (107) und die Öffnungen (131) der Anodenelektrode (111) zueinander ausgerichtet sind;
ein Anodenkammersubstrat (113) mit einem Hohlraum, welcher die Anodenkammer (115) bildet, wobei das Anodenkammersubstrat (113) an dem Anodenelektrodenträgersubstrat (107) befestigt ist und die Anodenelektrode (111) bedeckt;
ein Kathodenkammersubstrat (117) umfassend einen Hohlraum, welcher die Kathodenkammer (119) bildet, wobei die obere Fläche (327) des festen Trägers (121) an der zweiten adhesiven Schicht (105) haftet; und
eine Kathodenelektrode (123), welche an dem Boden (125) der Kathodenkammer (119) befestigt ist.

12. Mikrofluidische Vorrichtung (100) nach Anspruch 11, wobei der feste Träger (121) aus dem Kathodenkammersubstrat (117) gebildet ist.

13. Mikrofluidische Vorrichtung (100) nach einem der Ansprüche 11 bis 12, wobei die erste adhesive Schicht (103) und die zweite adhesive Schicht (105) in der Lage sind, elektrischen Strom zu leiten.

14. Mikrofluidische Vorrichtung (100) nach einem der Ansprüche 11 bis 13, wobei die Anodenelektrodenträgersubstrate (107) und/oder das Kathodenkammersubstrat (117) aus Leiterplatten (PCBs) gebildet sind.

15. Verfahren zur Herstellung der mikrofluidischen Vorrichtung (100) nach einem der Ansprüche 1 bis 14, wobei das Verfahren umfasst
Herstellen eines Anodenelektrodenträgersubstrats (107) mit einer Vielzahl von Öffnungen (109), welche leiterförmig angeordnet sind, und einer Anodenelektrode (111) umfassend eine Vielzahl von Öffnungen (131), welche in der Form mit den Öffnungen (109) des Anodenelektrodenträgersubstrats (107) übereinstimmen und welche auf einer Seite des Anodenelektrodenträgersubstrats (107) befestigt ist, so dass die Öffnungen (109) des Anodenelektrodenträgersubstrats (107) und die Öffnungen (131) der Anodenelektrode (111) ausgerichtet sind;
Fixieren eines Anodenkammersubstrats (113) mit einem Hohlraum, welches die Anodenkammer (115) bildet, an dem Anodenelektrodenträgersubstrat (107) um die Anodenelektrode (111) zu bedecken und um eine Anodenkammer (115) zu bilden;
Erzeugen eines Kathodenkammersubstrats (117), welcher die Kathodenkammer (119) bildet, und des festen Trägers (121) und einer Kathodenelektrode (123), welche an dem Boden der Kathodenkammer (119) befestigt ist;
Bilden einer ersten adhesiven Schicht (103) und einer zweiten adhesiven Schicht (105) an den gegenüberliegenden Seiten einer Ionenaustauschmembran (101); und
Haften des Anodenelektrodenträgersubstrats (107) an die erste adhesive Schicht (103) und Haften des Kathodenkammersubstrats (117) an die zweite adhesive Schicht (105).

16. Verfahren nach Anspruch 15, wobei das Herstellen des Anodenelektrodenträgersubstrats (107) umfasst:
Aufbringen eines Photoresistfilms (505) auf eine metallbeschichtete Leiterplatte (PCB 501, 503);
Bestrahlen mit UV-Licht durch eine Maske mit einer Struktur, welche ausgebildet ist um die Vielzahl von Öffnungen (109, 131) des Anodenelektrodenträgersubstrats (107) und der Anodenelektrode (111) zu bilden, und Entwickeln der Struktur auf der Anodenelektrode (111), wodurch die Vielzahl von Öffnungen (131) der Anodenelektrode (111) gebildet werden;
Ätzen des Metalls (503) des belichteten metallbeschichteten PCBs (501, 503); und
Schneiden des belichteten PCB (501) aus dem metallbeschichteten PCB (501, 503), um die Öffnungen (119) des Anodenelektrodenträgersubstrats (107) zu bilden.

17. Verfahren nach Anspruch 15, wobei die Herstellung des Kathodenkammersubstrats (117) umfasst:
Aufbringen eines Photoresistfilms (503) auf dem Kathodenkammersubstrat (601);
Belichten mit UV-Licht durch eine Maske mit einer Struktur, welches ausgebildet ist, um das feste Substrat (121) zu bilden, und Entwickeln der Struktur auf dem Kathodenkammersubstrat (601);
Ätzen des belichteten Kathodenkammersubstrats (601), wodurch das feste Substrat (121) und der Boden (125) der Kathodenkammer (119) gebildet wird;
Dampfbeschichten wenigstens eines Metalls (607, 609) auf dem Photoresistfilm (603) auf den Oberflächen (327) des festen Substrats (121) und auf dem Boden (125) der Kathodenkammer (119) durch Vakuumverdampfung umfassend Erwärmen mit einem Elektrodenstrahl;
Entfernen des Photoresistfilms (603) von den Oberflächen (327) des festen Substrats (121); und
Elektroplattieren des metallbeschichteten (607, 609) Bodens (125) des Kathodenkammersubstrats (117) mit einem anderen Metall (611).

18. Verfahren nach Anspruch 17, wobei das Herstellen des Kathodenkammersubstrats (117) des Weiteren das Beschichten der Oberfläche des festen Trägers (121) mit einer zellenbindenden Substanz umfasst.

19. Verfahren nach Anspruch 15, wobei das Bilden der ersten (103) und zweiten adhesiven Schichten (105) umfasst:
Aufschleudern eines Klebstoffes auf die jeweiligen Seiten der Ionenaustauschmembran (101);
Haften einer Seite der Ionenaustauschmembran, welche die mit der ersten adhesiven Schicht (103) beschichtete Seite ist, an das Anodenträgersubstrat (107), während die Ionenaustauschmembran (101) unter Verwendung einer Walze ausgebreitet wird; und
Binden der anderen Seite der Ionenaustauschmembran, welche die mit der zweiten adhesiven Schicht (105) beschichtete Seite ist, an das Kathodenkammersubstrat (117).

20. Verfahren nach Anspruch 15, wobei der Klebstoff bei Raumtemperatur flüssig ist und Haftvermögen aufweist, während er gehärtet ist, wenn Wärme oder UV-Licht auf den Klebstoff angewandt werden.

21. Verfahren nach Anspruch 15, wobei das Haften der ersten (103) und der zweiten adhesiven Schichten (105) an das Anodenelektrodenträgersubstrat (107) und an das Kathodenkammersubstrat (117) durchgeführt wird, indem das Anodenelektrodenträgersubstrat (107), die Ionenaustauschmembran (101) und das Kathodenkammersubstrat (117) in einer Reihe angeordnet werden und Ausüben von Druck auf die Reihe.

22. Verfahren zum Konzentrieren von zellen- oder virenhaltigen Zellen aus einer Probe, , und zum Lysieren der Zellen oder Viren unter Verwendung der mikrofluidischen Vorrichtung (100) nach einem der Ansprüche 1 bis 14, wobei das Verfahren umfasst
Einführung einer Lösung enthaltend Ionen mit einem Standardoxidationspotential, welches höher oder niedriger ist als das von Wasser, in die Anodenkammer (115) der mikrofluidischen Vorrichtung (100);
Einführen einer zellen- oder virenhaltigen Lösung und Ionen mit einem niedrigeren Standardreduktionspotential als das von Wasser in die Kathodenkammer (119) der mikrofluidischen Vorrichtung (100); und
Anlegen von elektrischem Strom über eine Anodenelektrode (111) und eine Kathodenelektrode (123), wodurch die Elektrolyse in der Anodenkammer (115) und der Kathodenkammer (119) induziert wird und Einstellen des pH-Wertes der Lösung in der Anodenkammer (115) und/oder der Kathodenkammer (119).

23. Verfahren nach Anspruch 22, wobei die Ionen, die in die Anodenkammer (115) eingeführt werden und welche ein niedrigeres Standardoxidationspotential aufweisen als das von Wasser, wenigstens ein Ion umfasst, gewählt aus der Gruppe bestehend aus NO₃⁻, F⁻, SO₄²⁻, PO₄³⁻ und CO₃²⁻.

24. Verfahren nach Anspruch 22, wobei die Ionen, die in die Anodenkammer (115) eingeführt werden und ein höheres Standardoxidationspotential als das vom Wasser aufweisen, Clumfassen.

25. Verfahren nach Anspruch 22, wobei die Ionen, die in die Kathodenkammer eingeführt werden, wenigstens ein Ion umfassen gewählt aus der Gruppe bestehend aus Na⁺, K⁺, Ca²⁺, Mg²⁺ and Al³⁺.

26. Verfahren nach Anspruch 22, wobei der pH-Wert der Lösungen in der Anodenkammer (115) und der Kathodenkammer (119) eingestellt wird, indem die Richtung des angelegten elektrischen Stroms, die Intensität des angelegten elektrischen Stroms, die Dauer des angelegten elektrischen Stroms, die Breite der Elektrode oder die Dicke der lonenaustauschmembran (101) verändert wird.

## Revendications

1. Dispositif microfluidique (100) pour concentrer des cellules à partir d'un échantillon contenant des cellules ou des virus et pour lyser les cellules ou les virus, le dispositif comprenant
une chambre anodique (115) contenant une électrode anodique (111) ;
une chambre cathodique (119) contenant une électrode cathodique (123) ; et
une membrane échangeuse d'ions (101) séparant la chambre anodique (115) et la chambre cathodique (119),
dans lequel un support solide (121) apte à retenir des cellules ou des virus de l'échantillon contenant des cellules ou des virus est disposé dans la chambre cathodique (119), la surface du support solide (121) étant revêtue d'une substance de liaison cellulaire, et le support solide (121) ayant une structure choisie dans le groupe constitué par une structure plane, une structure de type pilier, une structure de type bille et une structure de type tamis.

2. Dispositif microfluidique (100) de la revendication 1, dans lequel la substance de liaison cellulaire est une substance hydrophobe ayant un angle de contact avec l'eau de 70° à 90°, ou une substance donneuse de charges.

3. Dispositif microfluidique (100) de la revendication 2, dans lequel la substance hydrophobe est choisie dans le groupe constitué par l'octadécyltrichlorosilane (OTS), le tridécafluorotétrahydrooctyltriméthoxysilane (DTS), le chlorure d'octadécyldiméthyl(3-triméthoxysilylpropyl)ammonium (OTC), le polyéthylèneiminetriméthoxysilane (PEIM) et les mélanges de ceux-ci.

4. Dispositif microfluidique (100) de n'importe lesquelles des revendications 1 à 3, dans lequel le support solide (121) a une structure de type pilier avec des piliers (121) s'étendant de façon pratiquement perpendiculaire à partir du fond (125) de la chambre cathodique (119).

5. Dispositif microfluidique (100) selon n'importe lesquelles des revendications 1 à 4, dans lequel la membrane échangeuse d'ions (101) conduit le courant électrique et est imperméable aux ions et/ou aux gaz qui sont générés par électrolyse dans la chambre anodique (115) et dans la chambre cathodique (119).

6. Dispositif microfluidique (100) selon n'importe lesquelles des revendications 1 à 5, dans lequel l'électrode cathodique (123) est formée d'un métal, qui est apte à absorber l'hydrogène gazeux.

7. Dispositif microfluidique (100) selon n'importe lesquelles des revendications 1 à 6, dans lequel l'électrode anodique (111) est formée d'un métal, qui a un potentiel d'oxydation standard supérieur à celui de l'eau et qui ne réagit pas avec l'eau.

8. Dispositif microfluidique (100) de la revendication 7, dans lequel l'électrode cathodique (123) est formée de palladium (Pd).

9. Dispositif microfluidique de la revendication 8, dans lequel l'électrode anodique (111) est formée d'un métal choisi dans le groupe constitué du cuivre (Cu), du plomb (Pb), de l'argent (Ag), du chrome (Cr), du titane (Ti), du nickel (Ni), du zinc (Zn), du fer (Fe) et de l'étain (Sn).

10. Dispositif microfluidique (100) selon n'importe lesquelles des revendications 1 à 9, dans lequel la chambre cathodique (119) et/ou la chambre anodique (115) comprennent une entrée (229) pour l'introduction d'une solution dans la chambre (115, 119) et une sortie (231) pour l'évacuation d'une solution hors de la chambre (115, 119).

11. Dispositif microfluidique (100) selon n'importe lesquelles des revendications 1 à 10, comprenant en outre
une première couche adhésive (103) formée sur un côté de la membrane échangeuse d'ions (101) ;
une seconde couche adhésive (105) formée sur l'autre côté de la membrane échangeuse d'ions (101) ;
un substrat (107) de support d'électrode anodique, doté d'une pluralité d'ouvertures (109) qui sont agencées en forme d'échelle, où le substrat (107) de support d'électrode anodique est adhérent à la première couche adhésive (103) et ;
une électrode anodique (111) comprenant une pluralité d'ouvertures (131) qui correspondent par leur forme aux ouvertures (109) du substrat (107) de support d'électrode anodique, où l'électrode anodique (111) est fixée sur le substrat (107) de support d'électrode anodique avec les ouvertures (109) du substrat (107) de support d'électrode anodique et les ouvertures (131) de l'électrode anodique (111) qui sont alignées ;
un substrat (113) de chambre anodique doté d'une cavité formant la chambre anodique (115), où le substrat (113) de chambre anodique est fixé au substrat (107) de support d'électrode anodique et recouvre l'électrode anodique (111) ;
un substrat (117) de chambre cathodique doté d'une cavité, qui forme la chambre anodique (119), où la surface supérieure (327) du support solide (121) est adhérente à la seconde couche adhésive (105) ; et
une électrode cathodique (123), qui est fixée sur le fond (125) de la chambre cathodique (119).

12. Dispositif microfluidique de la revendication 11, dans lequel le support solide (121) est formé du substrat (117) de chambre cathodique.

13. Dispositif microfluidique (100) selon n'importe lesquelles des revendications 11 à 12, dans lequel la première couche adhésive (103) et la seconde couche adhésive (105) sont aptes à conduire le courant électrique.

14. Dispositif microfluidique (100) selon n'importe lesquelles des revendications 11 à 13, dans lequel le substrat (107) de support d'électrode anodique et/ou le substrat (117) de chambre cathodique sont formés de cartes de circuits imprimés (PCB, pour « Printed Circuit Boards »).

15. Procédé de production du dispositif microfluidique (100) selon n'importe lesquelles des revendications 1 à 14, le procédé comprenant les étapes consistant à
produire un substrat (107) de support d'électrode anodique doté d'une pluralité d'ouvertures (109), qui sont agencées en forme d'échelle, et une électrode anodique (111) comprenant une pluralité d'ouvertures (131), qui correspondent par leur forme aux ouvertures (109) du substrat (107) de support d'électrode anodique, et qui est fixée sur un côté du substrat (107) de support d'électrode anodique avec les ouvertures (109) du substrat (107) de support d'électrode anodique et les ouvertures (131) de l'électrode anodique (111) qui sont alignées ;
fixer un substrat (113) de chambre anodique doté d'une cavité formant la chambre anodique (115), au substrat (107) de support d'électrode anodique afin de recouvrir l'électrode anodique (111) et de former une chambre anodique (115) ;
produire un substrat (117) de chambre cathodique pourvu d'une cavité, qui forme la chambre cathodique (119), et le support solide (121), et une électrode cathodique (123), qui est fixée sur le fond de la chambre cathodique (119) ;
former une première couche adhésive (103) et une seconde couche adhésive (105) sur les côtés respectifs d'une membrane échangeuse d'ions (101) ; et
faire adhérer le substrat (107) de support d'électrode anodique à la première couche adhésive (103), et faire adhérer le substrat (117) de chambre cathodique à la seconde couche adhésive (105).

16. Procédé de la revendication 15, dans lequel la production du substrat (107) de support d'électrode anodique comprend les étapes consistant à
appliquer un film de résine photosensible (505) sur une carte de circuit imprimé à revêtement métallique (PCB 501, 503) ;
irradier un rayonnement UV à travers un masque dont le motif est conçu pour former la pluralité d'ouvertures (109, 131) du substrat (107) de support d'électrode anodique et de l'électrode anodique (111), et développer le motif sur l'électrode anodique (111), formant ainsi la pluralité d'ouvertures (131) de l'électrode anodique (111) ;
graver le métal (503) de la PCB à revêtement métallique (501, 503) découverte ; et
découper la PCB (501) découverte de la PCB à revêtement métallique (501, 503) pour former les ouvertures (119) du substrat (107) de support d'électrode anodique.

17. Procédé de la revendication 15, dans lequel la production du substrat (117) de chambre cathodique comprend les étapes consistant à
appliquer un film de résine photosensible (503) sur le substrat (601) de chambre cathodique ;
irradier un rayonnement UV à travers un masque dont le motif est conçu pour former le substrat solide (121), et développer le motif sur le substrat (601) de chambre cathodique ;
graver le substrat (601) de chambre cathodique découvert, formant ainsi le substrat solide (121) et le fond (125) de la chambre cathodique (119) ;
déposer en phase vapeur au moins un métal (607, 609) sur la résine photosensible (603) sur les faces supérieures (327) du substrat solide (121) et sur le fond (125) de la chambre cathodique (119) par évaporation sous vide faisant intervenir un apport de chaleur avec un faisceau électronique ;
éliminer le film de résine photosensible (603) des faces supérieures (327) du substrat solide (121) ; et
effectuer un dépôt électrolytique sur le fond (125), ayant un dépôt de métal (607, 609), du substrat (117) de chambre cathodique avec un autre métal (611).

18. Procédé de la revendication 17, dans lequel la production du substrat (117) de chambre cathodique comprend en outre l'étape consistant à revêtir la surface du support solide (121) avec une substance de liaison cellulaire.

19. Procédé de la revendication 15, dans lequel la formation des première (103) et seconde (105) couches adhésives comprend les étapes consistant à
appliquer par centrifugation un adhésif sur les côtés respectifs de la membrane échangeuse d'ions (101) ;
faire adhérer un côté de la membrane échangeuse d'ions, ledit côté étant revêtu de la première couche adhésive (103), au substrat (107) de support d'anode pendant que la membrane échangeuse d'ions (101) est étalée à l'aide d'un rouleau ; et
coller l'autre côté de la membrane échangeuse d'ions, ledit côté étant revêtu de la seconde couche adhésive (105), au substrat (117) de chambre cathodique.

20. Procédé de la revendication 15, dans lequel l'adhésif est liquide à température ambiante et acquiert de l'adhésivité pendant qu'il durcit lorsque de la chaleur ou un rayonnement UV est appliqué sur l'adhésif.

21. Procédé de la revendication 15, dans lequel l'adhésion des première (103) et seconde (105) couches adhésives au substrat (107) de support d'électrode anodique et au substrat (117) de chambre cathodique, respectivement, est réalisée en plaçant le substrat (107) de support d'électrode anodique, la membrane échangeuse d'ions (101) et le substrat (117) de chambre cathodique en réseau, et en appliquant chaleur et pression sur le réseau.

22. Procédé pour concentrer des cellules à partir d'un échantillon contenant des cellules ou des virus et pour lyser les cellules ou les virus à l'aide du dispositif microfluidique (100) selon n'importe lesquelles des revendications 1 à 14, le procédé comprenant les étapes consistant à
introduire une solution contenant des ions ayant un potentiel d'oxydation standard supérieur ou inférieur à celui de l'eau, dans la chambre anodique (115) du dispositif microfluidique (100) ;
introduire une solution contenant des cellules ou des virus et des ions ayant un potentiel de réduction standard inférieur à celui de l'eau, dans la chambre cathodique (119) du dispositif microfluidique (100) ; et
appliquer un courant électrique à travers une électrode anodique (111) et une électrode cathodique (123), induisant ainsi une électrolyse dans la chambre anodique (115) et la chambre cathodique (119) et ajuster le pH de la solution dans la chambre anodique (115) et/ou la chambre cathodique (119).

23. Procédé de la revendication 22, dans lequel les ions qui sont introduits dans la chambre anodique (115) et qui ont un potentiel d'oxydation standard inférieur à celui de l'eau comprennent au moins un ion choisi dans le groupe constitué de NO₃⁻, F⁻, SO4²⁻, PO₄³⁻ et CO₃²⁻.

24. Procédé de la revendication 22, dans lequel les ions qui sont introduits dans la chambre anodique (115) et qui ont un potentiel d'oxydation standard supérieur à celui de l'eau comprennent Cl⁻.

25. Procédé de la revendication 22, dans lequel les ions qui sont introduits dans la chambre cathodique comprennent au moins un ion choisi dans le groupe constitué de Na⁺, K⁺, Ca²⁺, Mg²⁺ et Al³⁺.

26. Procédé de la revendication 22, dans lequel le pH des solutions dans la chambre anodique (115) et la chambre cathodique (119) est ajusté en faisant varier la direction du courant électrique appliqué, l'intensité du courant électrique appliqué, la durée du courant électrique appliqué, la largeur de l'électrode, ou l'épaisseur de la membrane échangeuse d'ions (101).
